# EUROPEAN PATENT APPLICATION

(11) **EP 3 928 698 A1**
(43) Date of publication of application: **29.12.2021**
(21) Application number: 20382551.8
(22) Date of filing: 23.06.2020
(51) Int. Cl.: A61B 5/15, A61B 5/151, A61B 5/157

(54) **DEVICE FOR DETECTING AT LEAST ONE SUBSTANCE IN THE BLOOD OF AN INDIVIDUAL**

(71) Applicant: Duran Serradell, Victor, 08017 Barcelona (ES)
(72) Inventor: Duran Serradell, Victor, 08017 Barcelona (ES)
(74) Representative: Durán-Corretjer, S.L.P.

(57) **Abstract**

The invention relates to a device (1) for detecting at least one substance in the blood of an individual, which comprises a main body (10) and a replaceable sterile head (20), said replaceable head (20) comprising:
- means (32) for disinfecting the skin of said individual;
- means for collecting blood from said individual;
- means for detecting substances in the blood, wherein said means is a lateral flow test (23) and wherein the replaceable head (20) comprises means (31) for diluting the blood in order to perform the lateral flow test and in which the replaceable head (20) is functionally connected to the main body (10) of the device (1).

## Description

The present invention relates to a device for detecting at least one substance in the blood of an individual and to a replaceable head for use with the aforementioned device.

There are many medical reasons for obtaining blood samples from an individual to subsequently test them in order to detect substances in the aforementioned blood sample, such as collecting a blood sample in order to measure glucose levels in patients with diabetes or to detect antigens or antibodies associated with an infection.

In the known prior art, when seeking to detect a substance in the blood of an individual by means of a lateral flow test, the region of the skin from which the blood is to be drawn must first be disinfected using a disinfectant. In general, the skin should be punctured later using an appropriate device in order to obtain a blood sample. After the blood sample is obtained, an immunochromatographic strip is usually placed in contact with said blood sample. As the blood sample is usually not of sufficient volume, it is sometimes necessary to place the aforementioned strip in contact with a blood dilution solution in order to be able to carry out the lateral flow test.

Typically, the performance of a lateral flow test for detecting one or more substances in the blood of an individual requires several elements to be arranged separately, which has certain drawbacks, such as how easy it is to lose some of the elements, resulting in the inability to perform the test. Therefore, there is a need for devices that have all the necessary components to carry out all the steps of the detection, from disinfection of the blood collection site to the performance of the lateral flow test. The present invention solves this problem by disclosing a device for detecting at least one substance in the blood of an individual, which comprises all the elements necessary for performing the test and detecting the at least one substance.

Not only does the device of the present invention have all the necessary components in the device itself, it also has the advantage of having a replaceable head, which can be sterile and which contains elements that can be disposable, thus avoiding possible contamination between tests.

Another advantage of the device of the present invention is that it is not necessary to wait for the 10-15 min that these types of tests generally take, since once the blood and the dilution solution are placed in contact with the strip, said head can be separated from the main body to wait for the result of the test. Therefore, a new head can be placed in order to perform another test, which may or may not be the same, thus increasing the number of tests that can be performed in the same period of time, which is vitally important, for example when medical services are very busy.

An additional advantage of the device of the present invention is that it is not limited to one type of lateral flow test, but can be applied to any such type of test that is commercially available or may be developed in the future.

Therefore, in a first aspect, the present invention discloses a device for detecting at least one substance in the blood of an individual, which comprises a main body and a replaceable sterile head, said replaceable head comprising:
- means for disinfecting the skin of said individual;
- means for collecting blood from said individual;
- means for detecting substances in the blood, in which said means is a lateral flow test;
in which the replaceable head comprises means for diluting the blood in order to perform the lateral flow test and in which said replaceable head is functionally connected to the main body of the device.

Preferably, the aforementioned replaceable sterile head is disposable, i.e. it is intended to be used only once for each test, and must be replaced with a new sterile head in order to perform a new test on the same or another person.

The fact that the replaceable head comprises the means for disinfecting the skin of the individual, the means for collecting the blood from the individual, and the means for detecting substances in the blood allows the device that is the subject of the present invention to be used with more than one person or more than once by the same person, simply by replacing the used head with a new one, a task which can be performed quickly and easily by any user, without requiring any specific training. Among other advantages, this entails a reduction in the economic cost of carrying out multiple tests, since it is only necessary for the user, health professional, etc. to purchase the main body and the components contained therein once, and replaceable heads can be purchased for use with the previously purchased main body. This cost reduction is also associated with ensuring the safety of the individual to be tested, since the head comprises single-use elements, either for hygienic reasons or because they are single-use consumables.

Another advantage of the replaceable heads of the device object of the present invention is that it makes it possible to have several types of them, i.e. it makes it possible to have a plurality of different heads, each one of which is valid for detecting different substances. It is also possible to have different types of heads with, for example, different puncture depths, different materials, etc. In this case, the user or healthcare professional or medical facility can buy and use the heads that they need or are interested in and use them with a previously purchased main body.

The means for skin disinfection can be any antiseptic or disinfection means known to a person skilled in the art. In the context of the present invention, as it concerns the disinfection of living tissue, the term antiseptic is commonly used. Among the antiseptics that can be used in the present invention are ethyl alcohol, hydrogen peroxide, quaternary ammonium compounds, iodine, chlorhexidine, boric acid, among others. Preferably, the means for skin disinfection is a hydroalcoholic gel.

Advantageously, said means for collecting blood may comprise a lancet for puncturing the skin of said individual.

Preferably, said lateral flow test is a lateral flow immunoassay.

Preferably, said lateral flow test comprises an immunochromatographic strip, the strip and the lancet being housed in the same body in such a way that the lancet projects beyond the aforementioned strip.

Advantageously, the substance to be detected in human blood is selected from the group that comprises immunoglobulins, antigens, coagulation factors, albumin, glucose, proteins, vitamins, hormones, cytokines, among others.

Preferably, said main body comprises means for actuating the means for collecting blood. Advantageously, said means for actuating the means for collecting blood comprise two push buttons arranged opposite one another on the main body.

Preferably, the main body defines a longitudinal axis and the means for actuating the means for collecting blood are configured to move the means for collecting blood and the means for detecting substances in the blood in the direction of the aforementioned longitudinal axis in such a way that they project from the head through an opening therein. Advantageously, the means for actuating the means for collecting blood additionally comprise a cylinder joined to a spring, configured so that the cylinder pushes the means for collecting blood and the means for detecting substances in the blood. Preferably, the head comprises a spring for returning the means for collecting blood and the means for detecting substances in the blood into the body thereof.

Advantageously, the main body comprises a plunger coupled to a rod, said plunger being slidably coupled inside the main body in order to form a vacuum chamber and the means for detecting substances in the blood are in fluid communication with the vacuum chamber.

Preferably, the means for actuating the means for collecting blood are also configured to retain the plunger.

Advantageously, the plunger is actuated by a spring configured to move from an extended position to an inoperative position after the release of the plunger.

Advantageously, the means for diluting the blood are comprised in a cap of the replaceable sterile head.

Preferably, the replaceable head is made at least partially of a transparent material. Advantageously, the body that comprises the immunochromatographic strip and the lancet is made at least partially of a transparent material.

Preferably, the replaceable head is joined to the main body via a threaded joint. Alternatively, the replaceable head is joined to the main body via a bayonet joint or by dimensional interference, among others.

According to another aspect of the present invention, a replaceable head for use with a device according to the present invention is also disclosed.

According to another aspect of the present invention, a device for detecting at least one substance in the blood of an individual is also disclosed, comprising:
- means for collecting blood from said individual, which comprise a lancet for puncturing the skin of said individual,
- means for detecting substances in the blood, which comprise an immunochromatographic strip, wherein the lancet and the immunochromatographic strip are housed in the same body.

The terms "replaceable sterile head" and "replaceable head" are used equivalently and interchangeably throughout the present document. In this document, the directions horizontal, vertical, up, down, etc. are understood to be according to the working position of the device and the head that are subjects of the present invention in a position with the longitudinal axis thereof perpendicular to the ground.

A series of drawings of an embodiment of a device for detecting at least one substance in the blood of an individual object of the present invention are appended to ensure better understanding through explanatory but non-exhaustive examples.
- Fig. 1 is a perspective view of an exemplary embodiment of a device for detecting at least one substance in the blood of an individual according to the present invention.
- Fig. 2 is an exploded perspective view of the exemplary embodiment of Fig. 1.
- Fig. 3 is a front elevation view of the exemplary embodiment of Fig. 1.
- Fig. 4 is a cross-sectional view along the cut line IV-IV of Fig. 3.
- Fig. 5 is a front elevation view of the plunger of the exemplary embodiment of Fig. 1.
- Fig. 6 is a cross-sectional view along the cut line VI-VI of Fig. 5.
- Fig. 7 is a front elevation view and a bottom view of the body that comprises a lancet and an immunochromatographic strip of the exemplary embodiment of Fig. 1.
- Fig. 8 is a cross-sectional view along the cut line VIII-VIII of Fig. 7.
- Fig. 9 is a front elevation view and a bottom view of the cap of the exemplary embodiment of Fig. 1.
- Fig. 10 is a cross-sectional view along the cut line X-X of Fig. 9.
- Fig. 11 is a cross-sectional perspective view showing the removal of the cap of the exemplary embodiment of Fig. 1.
- Fig. 12 is a cross-sectional perspective view showing the operation of the means for actuating the blood collection means of the exemplary embodiment of Fig. 1.
- Fig. 13 is a cross-sectional perspective view showing the body that comprises a lancet and an immunochromatographic strip and the plunger and the rod thereof of the exemplary embodiment of Fig. 1, in an operative position.
- Fig. 14 is a detailed cross-sectional view of the means for actuating the blood collection means of the exemplary embodiment of Fig. 1, at the time when the aforementioned means are actuated.
- Fig. 15 is a detailed view of Fig. 14 when the user stops actuating the blood collection means.
- Fig. 16 is a cross-sectional perspective view showing the reinsertion of the cap in the exemplary embodiment of Fig. 1.
- Fig. 17 is a cross-sectional view of a cap in the position in which the dissolution liquid comes into contact with the lancet and the immunochromatographic strip of the exemplary embodiment of Fig. 1

In the figures, the same or equivalent elements have been identified with identical numerals.

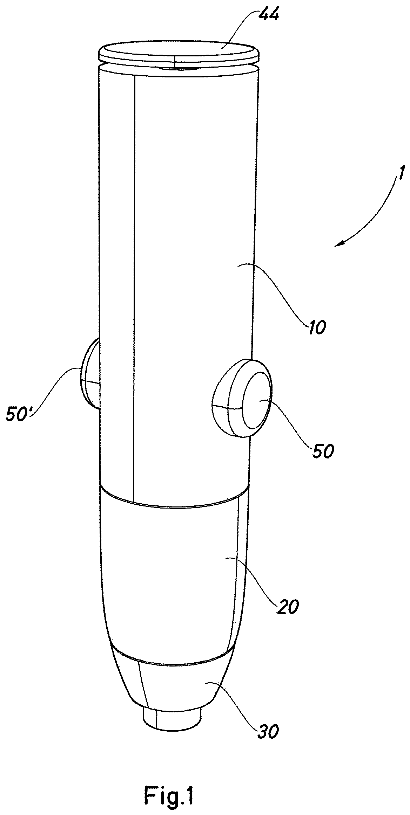

Fig. 1 shows a perspective view of an exemplary embodiment of a device for detecting at least one substance in the blood of an individual according to the present invention. This view shows the main body 10 of the device 1 joined by the joining end thereof to the joining end of the replaceable sterile head 20. Said replaceable head 20 comprises a cap 30 at the puncturing end thereof.

Two push buttons 50, 50' project from the main body 10 of the device 1, which in this exemplary embodiment is substantially cylindrical, arranged opposite one another thereon. Said push buttons 50, 50' are part of the means for actuating the puncturing means of the device 1, the operation of which is described in detail below.

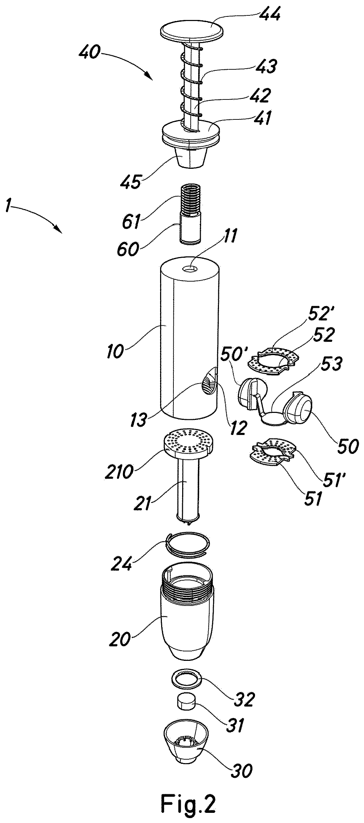

This figure also shows how, in the exemplary embodiment shown, the flange or handle 44 of the plunger 40 (see, among others, Fig. 2) projects from the main body 10 at the end thereof opposite the joining end.

Fig. 2 is an exploded perspective view of an exemplary embodiment of a device according to the present invention, which displays the internal components thereof. This figure shows the plunger 40, which comprises a head 41 joined to a rod 42 which in turn is joined to a flange or handle 44. The head 41 of the plunger 40 comprises a projecting portion 45 which, in this exemplary embodiment, has a substantially inverted frustoconical shape. This exploded view also shows the spring 43 that is responsible for actuating the plunger 40.

In this exploded view, the cylinder 60 and its spring 61, which are part of the means for actuating the blood collection means of the device 1, can be seen below the plunger 40.

In this exemplary embodiment, the main body 10 of the device 1 comprises at the free end thereof, i.e. at the end thereof opposite the joining end, the opening 11 through which the rod 42 of the plunger 40 passes when the device 1 is assembled. At the bottom of the aforementioned main body 10, near the joining end thereof, are the openings 13 facing one another (only one of them is visible due to the perspective used) through which the push buttons 50, 50' of the means for actuating the blood collection means of the device 1 project. Said push buttons 50, 50' are joined by a flexible joining part 53. Next to the push buttons 50, 50' are depicted the pairs of plates 51, 51', 52, 52' which are also comprised in the means for actuating the blood collection means of the device 1.

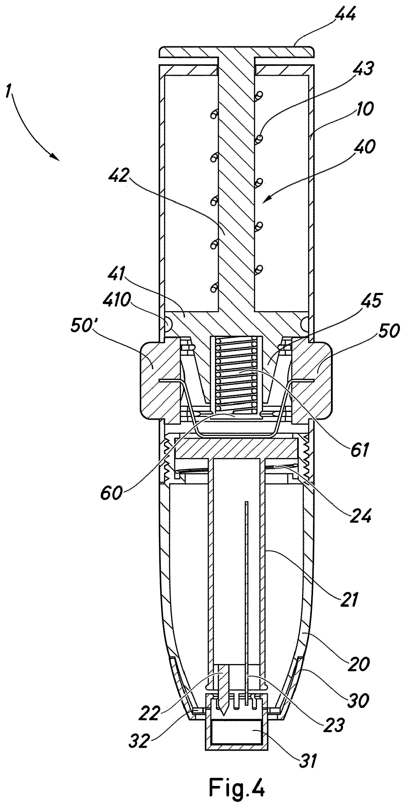

In the exemplary embodiment shown, the body 21 comprises at the puncturing end thereof a lancet 22 of the means for collecting the blood of an individual and an immunochromatographic strip 23 of the means for detecting substances in the blood of the device 1 (see e.g. Fig. 4). In the exemplary embodiment shown, the body 21 is substantially cylindrical and is joined by the end thereof opposite the puncturing end to a substantially circular base 210.

The exploded view of Fig. 2 also shows the spring 24 responsible for returning the body 21, and more specifically the lancet 22 and the immunochromatographic strip 23, into the replaceable head 20.

The disinfectant product 32 and the means for diluting the blood or dilution liquid 31, which is contained in a breakable container, are housed inside the cap 20 of the replaceable head 30.

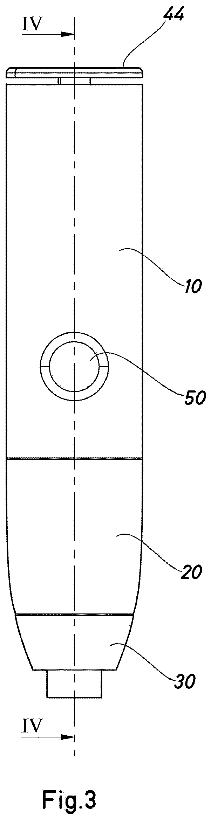

Fig. 3 is a front elevation view of an exemplary embodiment of a device according to the present invention. This figure shows the same elements as Fig. 1 and indicates the cut line IV-IV of the cross-sectional view of Fig. 4, which shows in detail how the internal components of the device 1 are distributed when the device is in the inoperative position, i.e. when it is ready to be used but before starting to collect the blood sample from the patient and the lateral flow analysis of the aforementioned sample.

As can be seen, in the exemplary embodiment shown, the main body 10 and the replaceable head 20 are joined via the respective joining ends thereof by means of a threaded joint. However, in other embodiments, said joint can be performed with other non-permanent or removable joining means, such as by dimensional interference.

This cross-sectional view of Fig. 4 clearly shows the location of the lancet 22 and the immunochromatographic strip 23 inside the body 21, as well as how such elements are protected by the cap 30 when it is placed on the puncturing end of the replaceable body 20. In the exemplary embodiment shown, both the body 21 and the replaceable head 20 are made of transparent materials, so that the user, medical professional, etc. can read the result of the lateral flow test without needing to remove the head. In other embodiments, the body 21 and the replaceable head 20 only contain a window of transparent material, i.e. they are only partially made of transparent material. If the body 21 and the replaceable head 20 comprise transparent windows, they must both be aligned so that the immunochromatographic strip 23 can be viewed from the outside of the replaceable head 20. In other embodiments, the body 21 and the replaceable head 20, or the windows thereof, instead of being made of transparent material, can be made of translucent material, the opacity thereof being suitable for inspecting the immunochromatographic strip 23.

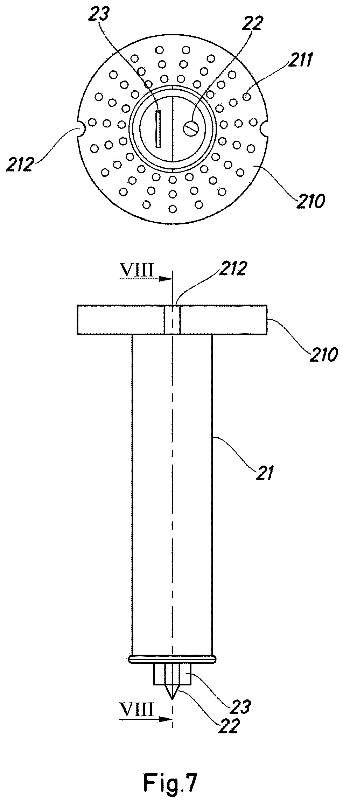

The spring 43 is joined by one of the ends thereof to the inside of the upper or free end of the main body 10 and to the head 41 of the plunger 40 by the other end thereof. As can be seen, in the inoperative position shown in Fig. 4, the spring 43 is in an elongated position, so that when the plunger 40 is released after pressing the push buttons 50, 50', the spring 43 returns to its inoperative or equilibrium position thereof, pulling the plunger 40, and more specifically its head 41, in such a way that a vacuum chamber is formed inside the main body 10. Said vacuum chamber is in fluid communication with the means for detecting substances in the blood, in this case, the immunochromatographic strip 23, through the openings 211 of the base 210 of the body 21 (for more details see Fig. 7).

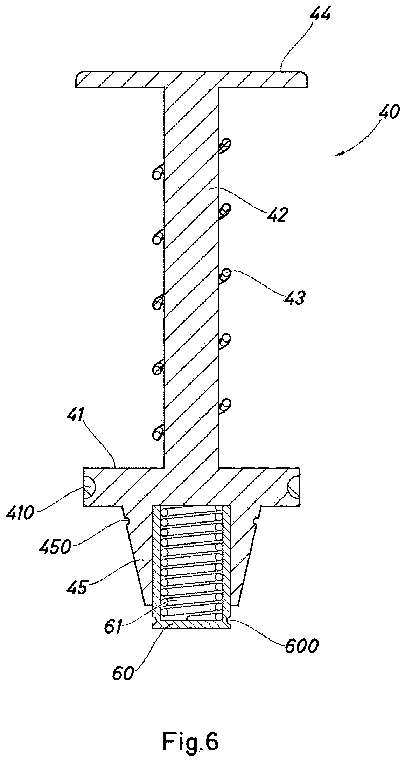

Fig. 6 clearly shows the recess 410 with a substantially semi-circular section in the head 41 of the plunger. Said recess is perimetral, i.e. it is present on the entire perimeter of the head 41. In the exemplary embodiment shown, said head 41 comprises a substantially frustoconical projecting portion 45, which in turn comprises a recess at the centre thereof which acts as a housing for the spring 61 and the cylinder 60.

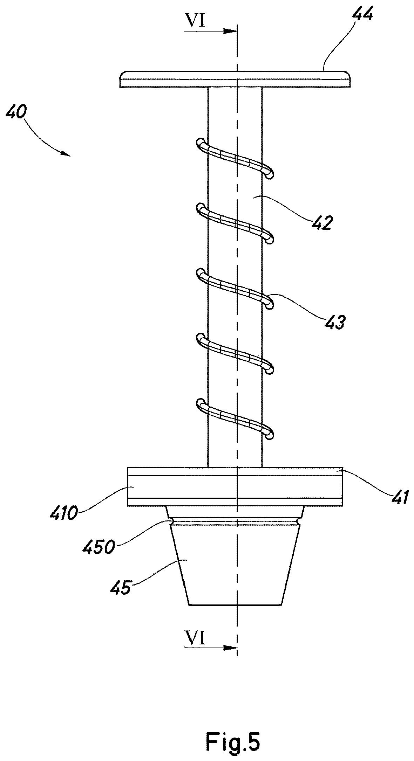

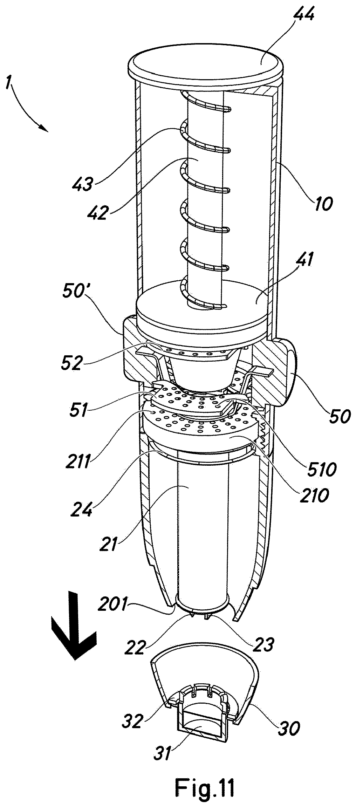

The piston 40 and the components thereof can be seen in greater detail in Fig. 5, which is a front elevation view of the plunger 40 of an exemplary embodiment of a device 1 according to the present invention. In addition to the elements of the plunger 40 described above, this view shows the perimetral recess or groove 450. Said perimetral recess 450 is configured so that the pair of plates 52, 52' (see e.g. Fig. 11 to 15) engage with said recess and hold it in the inoperative position of the device 1, preventing the plunger from moving along the longitudinal axis of the device 1, and more specifically, preventing the spring 43 from returning to its equilibrium or inoperative position thereof, pulling with it the head 41.

Fig. 5 also shows the cut line VI-VI of the cross-sectional view of Fig. 6 which, among other details, clearly shows how the spring 61 and the cylinder 60 are housed inside the central recess of the projecting portion 45. The piston 40 in Fig. 5 and 6 has been depicted in the inoperative position, which is the position prior to starting the process for detecting at least one substance in the blood of an individual. In this position, the spring 61 is in a compressed position. The cylinder 60 joined to the spring 61 has, at its proximal end to the body 21 (see e.g. Fig. 4), a perimetral recess 600 configured so that the pair of plates 51, 51' (see e.g. Fig. 11 to 15) engage with said recess and hold the cylinder 60 and the spring 61 in the compressed position until the user or operator actuates the buttons or push buttons 50, 50' (see e.g. Fig. 4).

Fig. 7 is a front elevation view and a bottom view of a body 21 comprising a lancet 22 and an immunochromatographic strip 23 of an exemplary embodiment of a device 1 according to the present invention. The lancet 22 is part of the means for collecting blood and the immunochromatographic strip 23 is part of the means for detecting substances in the blood. As can be seen in the front view, in the exemplary embodiment shown, the lancet 22 projects beyond the immunochromatographic strip 23, so that when the lancet 22 is inserted into the skin of the individual by performing an incision therein, the immunochromatographic strip 23 does not impact, or the impact is smaller, against the skin of the person undergoing the test to detect at least one substance, thus reducing the risk of damaging the immunochromatographic strip 23. However, in other embodiments, the ends of the strip 23 and the lancet 22 that project from the body 21 may be at the same level.

In the exemplary embodiment shown, the base 210 of the body 21, located at the end opposite the puncturing end of the aforementioned body 21, comprises two diametrically opposed recesses 212 that correspond to projecting portions with a matching shape located inside the replaceable head 20 so that they act as a guide for the aforementioned body 21 when it travels longitudinally through the replaceable head 20. In the case shown, the aforementioned recesses 212 are substantially semicircular. The base 210 can also comprise a plurality of through-openings 211 for placing the inside of the main body 10, and more specifically, the vacuum chamber formed by the plunger 40 and the main body 10, in fluid communication with the replaceable head 20, so as to create a negative pressure that can suck up the blood exiting the incision performed by the lancet 22 and lead said blood to the immunochromatographic strip 23. In the exemplary embodiment shown, the base 210 only comprises openings 211 in the circular crown defined by the outer edge of the base 210 and the cylinder of the body 21 (see exploded view of Fig. 2). However, in other embodiments the openings 211 can be present in the entire base 210, including the central region.

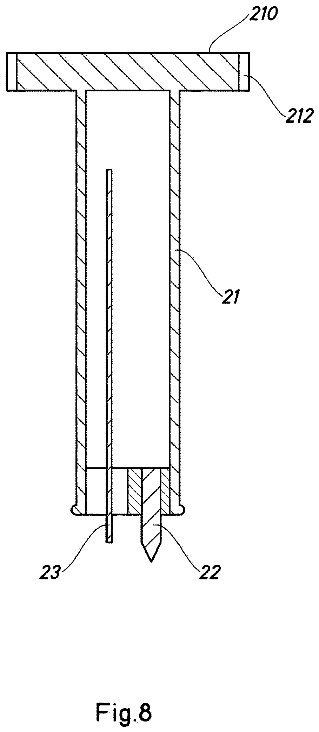

Fig. 7 also shows the cut line VIII-VIII of the cross-sectional view of Fig. 8, which shows the location of the lancet 22 and the immunochromatographic strip 23 inside the body 21. Said body 21, in the exemplary embodiment shown, is made of a transparent material that makes it possible to observe the results of the lateral flow test performed with the immunochromatographic strip 23 without the need to break the body 21 or remove the immunochromatographic strip 23 from the body. In cases where the body 21 is not made at least partially of a transparent or sufficiently translucent material, it is necessary to remove the immunochromatographic strip 23 from inside the body.

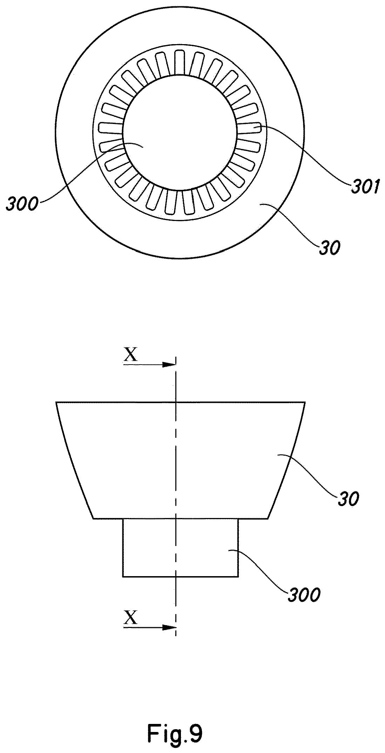

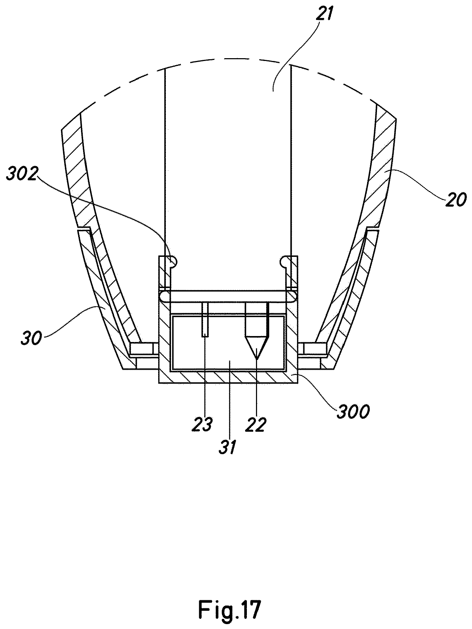

Fig. 9 is a front elevation view and a bottom view of a cap 30 of an exemplary embodiment of a device according to the present invention. In the exemplary embodiment shown, the cap 30 is configured to be attached to the puncturing end of the replaceable head 20 by dimensional interference (see e.g. Fig. 17). In other embodiments, the cap 30 can be joined to the replaceable head 20 by other types of non-permanent connections, e.g. threaded connections.

The cap 30 shown comprises a projecting portion 300 joined to the body thereof via a plurality of breakable ribs 301, the operation of which is detailed below.

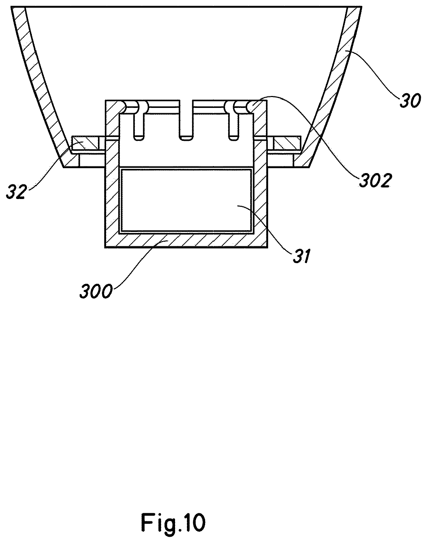

Fig. 9 shows the cut line X-X of the cross-sectional view of the cap 30 of Fig. 10. This cross-sectional view shows how the projecting portion 300 serves as a housing for a container that comprises the dilution liquid 31 for the lateral flow test for detecting substances in the blood. Said container is, preferably, breakable.

On the inside of the projecting portion 300 are the tabs 302. Around the base of the tabs 302 is a disc of disinfectant product 32 for disinfecting the region of the skin of the person undergoing the test to detect at least one substance in the blood, hereinafter the patient, where the incision is to be performed by means of the lancet 22.

Said disinfectant product 32 is housed inside the cap 30 in order to guarantee the sterility and hygiene thereof. To use it, the disc of disinfectant product 32 is removed and rubbed over the region of the skin of the patient on which the test is to be performed. Preferably, the aforementioned disinfectant product 32 is a disinfectant gel in solid or semi-solid state.

Fig. 11 to 13 show, in a perspective view of an exemplary embodiment of a device according to the present invention, partially in cross-section, the various steps of the process for detecting at least one substance in the blood of the patient. The fact that the device 1 is partially in cross-section in these figures makes it easier to appreciate the position of the internal components thereof in the various phases or steps of the process.

Fig. 11 shows the removal of the cap 30 from the device 1. The removal of the cap 30 initiates the process for detecting at least one substance in the blood of an individual. After removing the aforementioned cap, the user or person responsible for performing the test can access the disinfectant product 32 to disinfect the region of the skin of the patient where the incision will be performed with the lancet 22.

When the aforementioned cap 30 is removed, it must be kept, as it will be needed later.

The arrow shown in Fig. 11 illustrates the removal of the cap 30 by pulling on the cap in an axial direction, i.e. following the direction defined by the longitudinal axis of the device 1. However, in other embodiments in which the means for joining the cap 30 and the replaceable head 20 are different from the ones that are shown herein, the movement to remove the cap 30 may also be different.

This view shows the opening 201 at the puncturing end of the replaceable head 20. In the exemplary embodiment shown, said opening 201 is circular, although in other embodiments it may have other shapes.

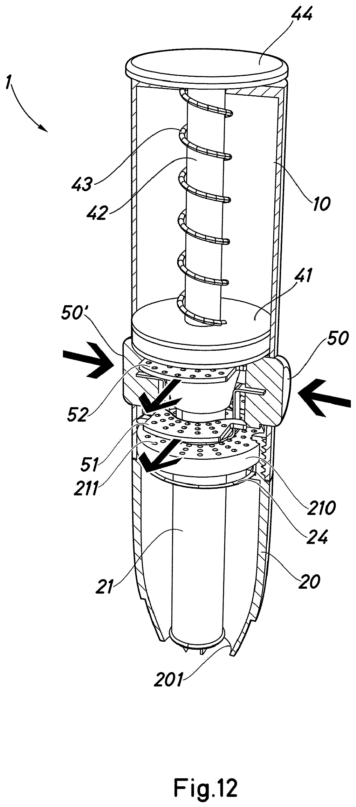

Fig. 12 shows the moment at which, after placing the puncturing end of the replaceable head 20 in contact with the region of the skin of the patient where the blood sample for the test is to be taken, the person responsible for performing the test presses or actuates the buttons or push buttons 50, 50'. When the buttons 50, 50' are pressed, the pairs of plates 51, 51', 52, 52' (due to the perspective used, plates 51' and 52' are hidden) retract, so that the plunger 40 and the cylinder 60 are released (see e.g. Fig. 4). This movement of the buttons 50, 50' and the pairs of plates 51, 51', 52, 52' is depicted by the corresponding arrows that can be seen in Fig. 12.

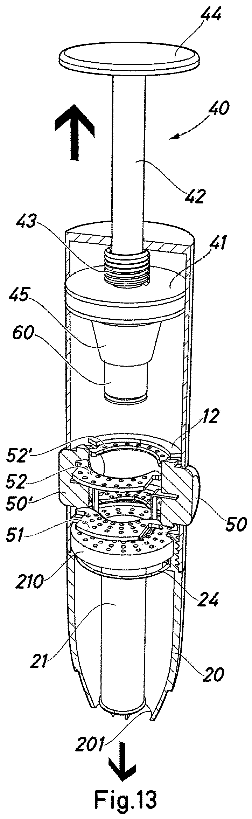

Fig. 13 shows the moment at which, after pressing the buttons 50, 50' and activating the means for actuating the blood collection means, the plunger 40 rises or moves towards the free end of the main body 10 and the body 21 lowers or moves towards the puncturing end of the replaceable head 20.

When the buttons 50, 50' are pressed, the plates 52, 52' retract and slide along the corresponding guides 12 inside the main body 10 until they exit the recess 450 of the projecting portion 45 of the plunger 40 and the latter is released, at which time the spring 43 returns to its equilibrium position and pulls the plunger 40 with it, thus creating a vacuum or negative pressure inside the main body 10.

At the same time as the plunger 40 is released, after pressing the buttons 50, 50' the body 21 is also activated or moved, since by pressing the two buttons 50, 50' the distance between them is shortened and the joining part 53 deforms so that the lower end or planar portion (see Fig. 2) descends and pushes the body 21 in an axial direction with respect to the longitudinal axis of the device 1. In addition, the device 1 of the exemplary embodiment shown comprises a cylinder 60 joined to a spring 61 and housed inside a central recess in the projecting portion 45 of the plunger, which is also released after pressing the buttons 50, 50'. When the cylinder 60 is released, it is pushed out because the spring 61, which was compressed, attempts to return to its equilibrium position. When it is pushed out, the cylinder 60 strikes the bottom or planar portion of the joining part 53 and helps the body 21 to descend quickly so that the lancet 22 can perform the skin incision correctly and reduce the pain caused to the patient. The striking of the cylinder 60 takes place at a higher speed than the speed at which the plunger 40 moves so that when the plunger rises or slides towards the free end of the main body 10, the cylinder 60 has already struck the joining part 53 and helped to perform the incision by the lancet 22 in the skin of the patient. As can be seen, after the cylinder 60 has been fired, it moves together with the spring 61 (in the picture it is hidden) with the plunger 40.

In order to release or fire the cylinder 60, the buttons 50, 50' also move the pair of plates 51, 51' which, in the inoperative position, engage with the recess 600 of the cylinder 60 (see Fig. 6), but when retracted actuated by the push buttons 50, 50' exit the aforementioned recess 600 and allow the cylinder 60 to be pushed out at high speed by the spring 61 that attempts to return to its equilibrium position.

As can be seen in figures 11 to 13, in the exemplary embodiment shown the pairs of plates 51, 51', 52, 52' comprise a plurality of through-openings distributed over the surface thereof. Said openings are configured to facilitate fluid communication between the replaceable head 20 and the vacuum chamber formed by the plunger 40 and the inside of the main body 10. However, in other embodiments, the aforementioned pairs of plates 51, 51', 52, 52' may have no openings.

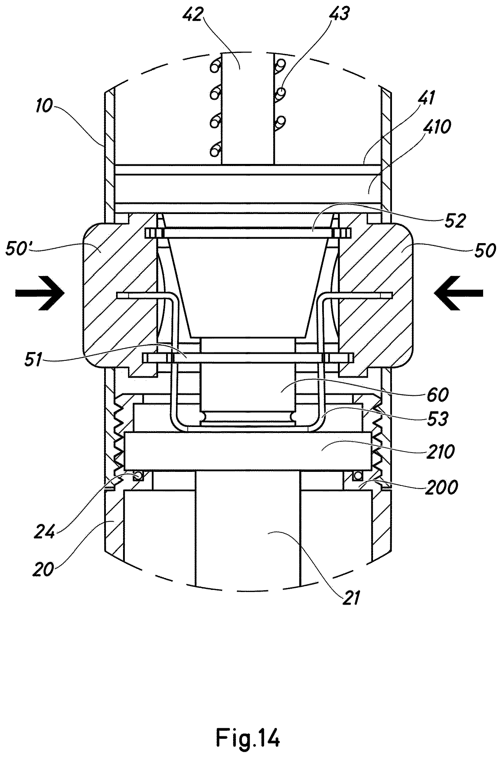

Fig. 14 shows, in cross-section, the detail of the pair of buttons 50, 50' and the other elements of the means for actuating the blood collection means of a device 1 at the moment at which the user presses the aforementioned pair of buttons 50, 50'. More specifically, Fig. 14 shows the moment at which the cylinder 60 strikes the joining part 53 and the base 210 of the body 21 so that the body moves towards the puncturing end of the replaceable head 20 until the base 210 abuts with the projecting portion 200, in which position the lancet 22 and the immunochromatographic strip 23 project from the replaceable head 20 through the opening 210, thus making it possible to puncture the skin of the patient. The projecting portion 200 is also responsible for housing the spring 24.

Shortly after the moment shown in Fig. 14, the plunger 40 begins to rise or move.

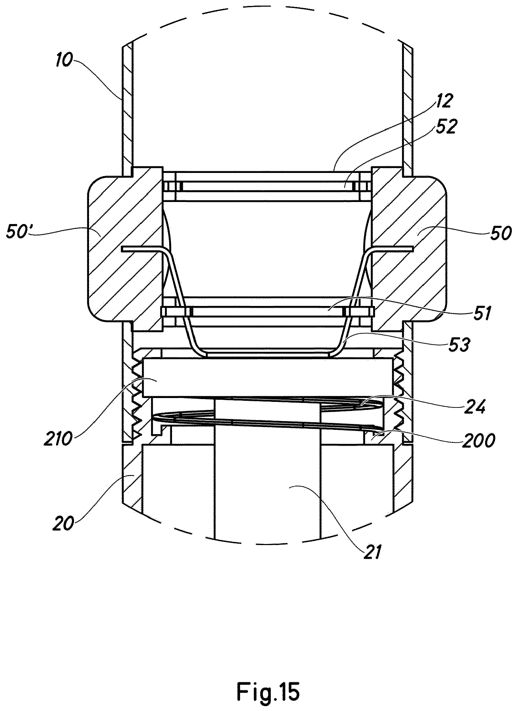

Fig. 15 is a detail view similar to that of Fig. 14, but showing the moment at which the user stops pressing or actuating the push buttons 50, 50'. As can be seen, when the actuating of the buttons 50, 50' is stopped, the body 21 retracts or moves in the opposite direction until returning to its initial or inoperative position in which the lancet 22 and the immunochromatographic strip 23 are protected inside the replaceable head 20. The replaceable head 20 in the exemplary embodiment shown comprises the spring 24 that is responsible for exerting a force towards the inside of the device, i.e. in the direction opposite to that exerted by the joining part 53 and the cylinder 60.

Once the body 21 returns to the initial position, and thus the lancet 22 and immunochromatographic strip 23 are back inside the replaceable head 20, it is possible to press the buttons 50, 50' again so that the joining part 53 moves or pushes the body 21 again and the lancet 22 and the immunochromatographic strip 23 project from the replaceable head 20 again. When the buttons 50, 50' are no longer being pressed, the body 21 returns to its initial position as described above. This operation can be repeated as many times as desired or necessary. These multiple punctures are considered to be part of the same test. These multiple punctures can be performed, for example, in the case that the first puncture was not satisfactory.

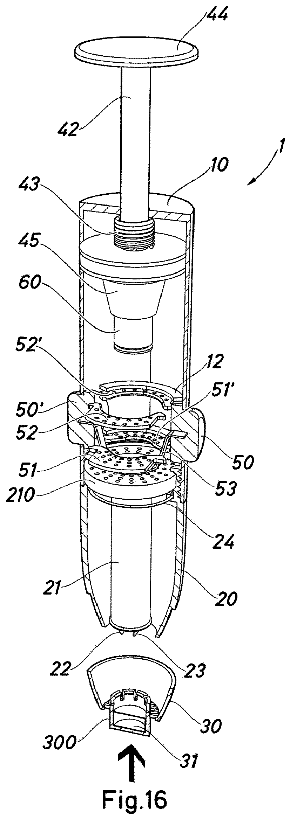

At the moment shown in Fig. 15, the plunger 40 is already in a position similar to that shown in Fig. 13 or Fig. 16.

Fig. 16 shows, in perspective view, the reinsertion of the cap 30 onto the device 1, which is shown partially in cross-section. This process is performed after the necessary blood sample has been obtained and after the immunochromatographic strip 23 has been placed in contact with the necessary amount of said blood sample.

When the immunochromatographic strip 23 is ready to perform the lateral flow test for detecting at least one substance in the blood sample obtained, the cap 30 is reinserted onto the puncturing end of the replaceable head 20. As can be seen, the aforementioned cap 30 continues to hold the container that contains the dilution liquid 21.

Once the cap 30 is in place, the person responsible for performing the test may proceed to press the projecting portion 300 of the cap 30 against the replaceable head 20, i.e. to press in the axial direction with respect to the longitudinal axis of the device 1 but in the direction opposite to the direction in which the body 21 travels. In order to unequivocally show the direction of the force to be exerted on the projecting portion 300 of the cap 30, an arrow is shown in Fig. 16.

If enough force is applied as indicated, the breakable ribs 301 break and the projecting portion 300 collapses, arriving at the situation shown in Fig. 17.

Fig. 17 is a detailed view of a section of the device 1 with the projecting portion 300 of the cap 30 collapsed or inserted towards the inside of the replaceable head 20. In this position, the container that contains the dilution liquid 21 breaks under the pressure exerted mainly by the lancet 22 and, to a lesser extent, by the immunochromatographic strip 23, so that the aforementioned immunochromatographic strip 23 comes into contact with the dilution liquid 21 and absorbs said liquid.

As discussed above, in the exemplary embodiment shown, the body 21 and the replaceable head 20 are made of a transparent material so that the result of the lateral flow test can be observed without having to dismantle the head 20 and/or body 21 in order to remove the immunochromatographic strip 23 and observe the test results.

In this exemplary embodiment and in the position shown in Fig. 17, the tabs 302 fit by dimensional interference with the body 21. Said body 21 may comprise a perimetral lip at the lower or puncturing end thereof in order to increase the dimensional interference with the tabs 302.

After the test, the replaceable head 20 can be removed from the main body 10, in this case by unscrewing, and the used replaceable head 20 can be replaced with a new one having the same or different characteristics. There can be several types of replaceable heads depending on, for example, the type of substance to be detected and/or the desired or necessary depth of puncture by the lancet 22.

It is also possible to remove the replaceable head 20 immediately after placing the immunochromatographic strip 23 in contact with the dilution liquid 31, thus reducing the waiting time between device operating cycles. In general, from the moment the immunochromatographic strip 23 comes into contact with the dilution liquid 31, a certain waiting period is required before the test results can be obtained. This wait can be done with the replaceable head removed from the device 1, which can be performing another test after being equipped with a new replaceable head 20.

Although not recommended, it is also possible to remove the replaceable head 20 before, or immediately after, placing the cap 30 on the puncturing end of the aforementioned head 20. In this case, the step of pressing the projecting portion 300 and placing the dilution liquid 31 in contact with the immunochromatographic strip 23 is carried out with the replaceable head 20 separated from the main body 10 of the device 1.

Although the invention has been presented and described in reference to its embodiments, it is understood that these have no limiting effect on the invention, so that multiple structural details or others that may be obvious for a person skilled in the art may vary after interpreting the subject matter that is disclosed in the present description, claims and drawings. In particular, in principle and unless explicitly stated otherwise, all the features of each of the different embodiments and alternatives shown and/or suggested can be combined with one another. Therefore, all the variants and equivalents will fall within the scope of the present invention if they can be considered to be comprised in the broader scope of the following claims.

## Claims

1. Device for detecting at least one substance in the blood of an individual, which comprises a main body and a replaceable sterile head, said replaceable head comprising:
- means for disinfecting the skin of said individual;
- means for collecting blood from said individual;
- means for detecting substances in the blood, wherein said means is a lateral flow test
**characterised in that** the replaceable head comprises means for diluting the blood in order to perform the lateral flow test and **in that** said replaceable head is functionally connected to the main body of the device.

2. Device according to claim 1, **characterised in that** said means for collecting blood comprise a lancet for puncturing the skin of said individual.

3. Device according to either claim 1 or claim 2, **characterised in that** said lateral flow test is a lateral flow immunoassay.

4. Device according to claims 2 and 3, **characterised in that** said lateral flow test comprises an immunochromatographic strip, the strip and the lancet being housed in the same body in such a way that the lancet projects beyond the aforementioned strip.

5. Device according to any of the preceding claims, **characterised in that** the substance to be detected in human blood is selected from the group that comprises immunoglobulins, antigens, coagulation factors, albumin, glucose, proteins, vitamins, hormones, cytokines, among others.

6. Device according to any of the preceding claims, **characterised in that** said main body comprises means for actuating the means for collecting blood.

7. Device according to claim 6, **characterised in that** said means for actuating the means for collecting blood comprise two push buttons arranged opposite one another on the main body.

8. Device according to either claim 6 or claim 7, **characterised in that** the main body defines a longitudinal axis and **in that** the means for actuating the means for collecting blood are configured to move the means for collecting blood and the means for detecting substances in the blood in the direction of the aforementioned longitudinal axis in such a way that they project from the head through an opening therein.

9. Device according to claim 8, **characterised in that** the means for actuating the means for collecting blood additionally comprise a cylinder joined to a spring, configured so that the cylinder pushes the means for collecting blood and the means for detecting substances in the blood.

10. Device according to either claim 8 or claim 9, **characterised in that** the head comprises a spring for returning the means for collecting blood and the means for detecting substances in the blood into the body thereof.

11. Device according to any of the preceding claims, **characterised in that** the main body comprises a plunger coupled to a rod, said plunger being slidably coupled inside the main body in order to form a vacuum chamber and **in that** the means for detecting substances in the blood are in fluid communication with the vacuum chamber.

12. Device according to any one of claims 6 to 8 and 9, **characterised in that** the means for actuating the means for collecting blood are also configured to retain the plunger.

13. Device according to any one of claims 10 to 12, **characterised in that** the plunger is actuated by a spring configured to move from an extended position to an inoperative position after the release of the plunger.

14. Device according to any of the preceding claims, **characterised in that** the means for diluting the blood are comprised in a cap of the replaceable sterile head.

15. Device according to any of the preceding claims, **characterised in that** the replaceable head is made at least partially of a transparent material.

16. Device according to claims 4 and 15, **characterised in that** the body that comprises the immunochromatographic strip and the lancet is made at least partially of a transparent material.

17. Device according to any of the preceding claims, **characterised in that** the replaceable head is joined to the main body via a threaded joint.

18. Replaceable head for use with a device according to any one of claims 1 to 17.
